(19) Europäisches Patentamt
European Patent Office
Office européen des brevets

(11) EP 4 454 647 A1

(12) **EUROPEAN PATENT APPLICATION**
published in accordance with Art. 153(4) EPC

(43) Date of publication:
**30.10.2024 Bulletin 2024/44**

(21) Application number: **22909620.1**

(22) Date of filing: **21.11.2022**

(51) International Patent Classification (IPC):
***A61K 31/4375*** *(2006.01)* ***A61P 35/00*** *(2006.01)*

(52) Cooperative Patent Classification (CPC):
**A61K 31/4375; A61P 35/00;** Y02P 20/55

(86) International application number:
**PCT/CN2022/133165**

(87) International publication number:
**WO 2023/116304 (29.06.2023 Gazette 2023/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.12.2021 CN 202111599362**

(71) Applicant: **Neurodawn Pharmaceutical Co., Ltd.**
**Jiangsu 211199 (CN)**

(72) Inventors:
- **ZHANG, Zhengping**
  **Nanjing, Jiangsu 210046 (CN)**
- **LI, Fulong**
  **Nanjing, Jiangsu 210046 (CN)**
- **WANG, Lei**
  **Nanjing, Jiangsu 210046 (CN)**
- **CHEN, Rong**
  **Nanjing, Jiangsu 210046 (CN)**
- **YANG, Weidong**
  **Nanjing, Jiangsu 210046 (CN)**
- **FANG, Fang**
  **Nanjing, Jiangsu 210046 (CN)**
- **AN, Wenji**
  **Nanjing, Jiangsu 210046 (CN)**
- **HUA, Yao**
  **Nanjing, Jiangsu 210046 (CN)**
- **FENG, Lin**
  **Nanjing, Jiangsu 210046 (CN)**

(74) Representative: **Lavoix**
**Bayerstraße 83**
**80335 München (DE)**

# (54) USE OF A CLASS OF 1,4-DIHYDRO-NAPHTHYRIDINE DERIVATIVES IN TREATMENT OF TUMORS

(57) The present invention relates to the use of a class of 1,4-dihydro-naphthyridine derivatives in the treatment of tumors.

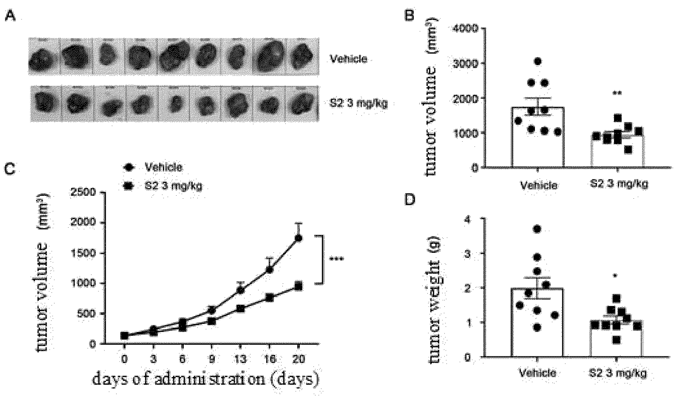


FIG.13



Processed by Luminess, 75001 PARIS (FR)

## Description

This application claims the priority of Chinese Patent Application No. 202111599362.3, filed with the China National Intellectual Property Administration on December 24, 2021, and titled with "USE OF A CLASS OF 1,4-DIHYDRO-NAPHTHYRIDINE DERIVATIVES IN TREATMENT OF TUMORS", which is hereby incorporated by reference in its entirety.

## FIELD

The present invention belongs to the field of pharmaceuticals, and relates to new use of a 1,4-dihydro-naphthyridine derivative, more particularly to use of a 1,4-dihydro-naphthyridine derivative in the manufacture of a medicament for treating a tumor. The tumor is a solid tumor, including nervous system tumors and non-nervous system tumors. The nervous system tumors include glioblastoma, neuroblastoma, etc., and the non-nervous system tumors include liver cancer, colon cancer, gastric cancer, etc.

## BACKGROUND

Glioblastoma (GBM) is the most common malignant primary intracranial tumor, accounting for approximately 57% of all gliomas and 48% of all primary central nervous system (CNS) malignant tumors (Neuro-oncology, 2018. 20: p. iv1-iv86). The mortality rate of glioblastoma is extremely high. In the United States, the 1-year survival rate of glioblastoma patients from 2000 to 2014 was 41.4%, and the 5-year survival rate was only 5.8% (CA: a cancer journal for clinicians, 2020. 70(4): p. 299-312). The cause of glioblastoma is still poorly understood; the only known possible causative factor is ionizing radiation. It also brings certain challenges to the prevention and treatment of glioblastoma.

According to the degree of malignancy, glioblastoma can be divided into grades I to IV. Among them, I and II are gliomas with low malignancy, and III and IV are gliomas with high malignancy. The treatment methods of glioblastoma mainly include surgical resection, radiotherapy, and systemic therapy (chemotherapy, targeted therapy). Among them, post-surgery temozolomide concurrent radiotherapy combined with adjuvant chemotherapy has become the standard regimen for newly diagnosed glioblastoma. In addition to temozolomide, the current main chemotherapy drugs include nitrosoureas, procarbazine, platinums, vinblastines, and camptothecins. However, the anti-tumor action mechanism of conventional chemotherapy drugs cannot meet today's clinical needs.

In eukaryotic cells, macropinocytosis is an important pathway for cells to internalize extracellular substances and dissolve molecules. The macropinocytosis process has a dual effect on tumor cells. In the case of nutrient deficiency, tumor cells can internalize extracellular nutrients through the macropinocytosis pathway, thereby promoting tumor cell growth. However, overactivation of the macropinocytosis process through drug treatment or abnormal expression of RAS gene can induce a new mode of death: methuosis. Methuosis is a new proteasome-independent form of cell death, characterized by the cytoplasm being filled with a large number of vacuoles derived from macropinocytosomes. This mode of death was first discovered in glioblastoma cells (Oncogene, 1999. 18(13): p. 2281-90). Further research found that this mode of death is triggered by changes in macropinocytosomes that are independent of clathrin, which eventually forms a large number of vacuoles, causing cell rupture and death (Journal of medicinal chemistry, 2012. 55(5): p. 1940-56). Current research has found that a variety of molecules are involved in the formation of macropinosomes, including Ras, Rac1, Arf6, Rab7, etc. (The American journal of pathology, 2014. 184(6): p. 1630-42). Rac1 can affect the formation of macropinocytosomes by regulating the assembly of actin. In addition, the activation of Rac1 can reduce activated Arf6 through the mediation of GIT-1, thereby hindering the circulation of macropinocytosomes (Molecular cancer research: MCR, 2010. 8(10): p. 1358-74). In recent years, it has been found that some small molecule compounds induce methuosis of cells by activating mechanisms such as MKK4, JNK and Rac1, including methamphetamine, indolyl chalcone, vaquinnol-1, MOMIPP, CX-5011, etc. (Oncotarget 2016, 7, 55863-55889; BMC cancer 2019, 19, 77; BBA-Molecular Cell Research 2020, 118807).

1, 4-dihydro-naphthyridine derivatives are compounds that can both inhibit acetylcholinesterase activity and block the influx of extracellular calcium ions into cells through calcium channels. By inhibiting the activity of cholinesterase, 1, 4-dihydro-naphthyridine derivatives can delay the hydrolysis rate of acetylcholine, increase the level of acetylcholine in the synaptic cleft, and achieve the effect of treating Alzheimer's disease and vascular dementia (Chinese Patent Nos. CN104203945B and CN106632317A). In addition, by inhibiting the influx of calcium ions, 1, 4-dihydro-naphthyridine derivatives can improve the tolerance of nerve cells to ischemia, expand cerebral blood vessels and improve cerebral blood supply, protect neurons, and effectively improve the cognitive function of patients with vascular dementia.

## SUMMARY

1, 4-dihydro-naphthyridine derivatives are compounds that can inhibit the activity of acetylcholinesterase and

block the influx of extracellular calcium ions into cells through calcium channels, which can be used to treat Alzheimer's disease and vascular dementia. Such compounds can induce methuosis of tumor cells through a new mechanism of action that does not rely on cholinesterase inhibition and calcium channel blocking, thereby exerting anti-tumor effects.

A purpose of the present invention is to provide a drug that can treat tumors through a new mechanism and effectively prolong the survival period of patients. The drug is a 1,4-dihydro-naphthyridine derivative or a pharmaceutically acceptable salt thereof.

In particular, the present invention provides use of a 1,4-dihydro-naphthyridine derivative represented by Formula I or a pharmaceutically acceptable salt thereof in the manufacture of a medicament for treating a tumor,

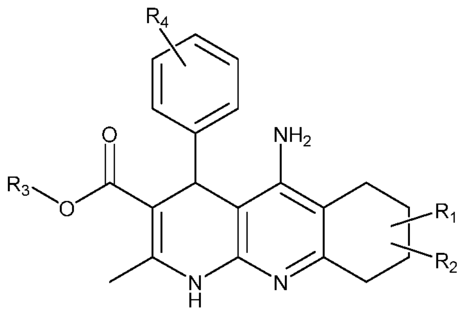

Formula I

wherein, $R_1$ or $R_2$ is hydrogen, halogen or $C_1$-$C_6$ alkyl;

$R_3$ is selected from $C_1$-$C_6$ alkyl, wherein any $-CH_2-$ in the alkyl may be replaced by one or more -O-;

$R_4$ is hydrogen or halogen.

In particular, the derivative is selected from the group consisting of:

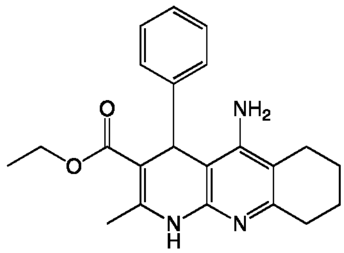

S1

Compound 1 represented by S1;

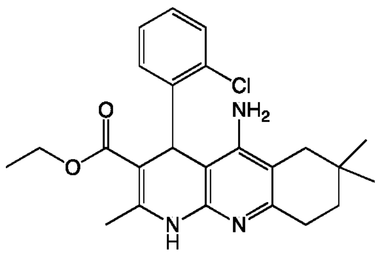

S2

Compound 2 represented by S2;

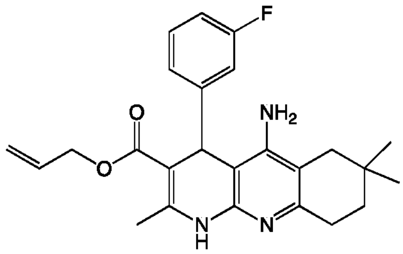

S3

Compound 3 represented by S3;

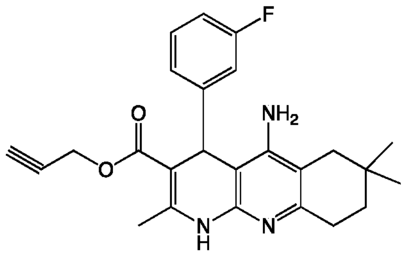

S4

Compound 4 represented by S4;

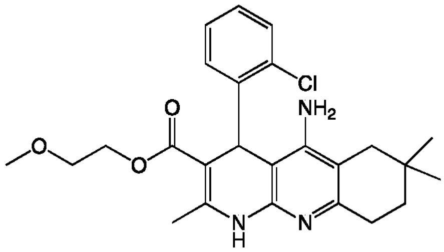

S5

Compound 5 represented by S5; and

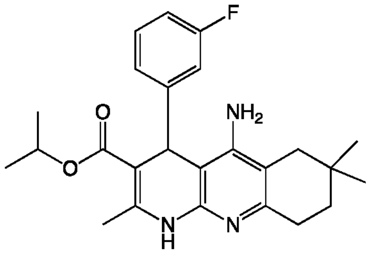

S6

Compound 6 represented by S6.

The above compounds induce methuosis of tumor cells, cause vacuolated cell phenotype, inhibit the proliferation of tumor cells, and exert a function of inhibiting tumor growth, ultimately achieving the purpose of treating tumors.

Therefore, the present invention provides new use of a 1,4-dihydro-naphthyridine derivative or a pharmaceutically acceptable salt thereof or a pharmaceutical composition thereof in the manufacture of a medicament for treating a tumor.

The tumor is a solid tumor, including nervous system tumors and non-nervous system tumors. The nervous system tumors include glioblastoma, neuroblastoma, etc., and the non-nervous system tumors include liver cancer,

colon cancer, gastric cancer, etc.

In particular, the present invention provides use of a 1,4-dihydro-naphthyridine derivative in the treatment of a solid tumor.

The present invention provides use of a 1,4-dihydro-naphthyridine derivative in the treatment of a nervous system tumor.

The present invention provides use of a 1,4-dihydro-naphthyridine derivative in the treatment of a brain tumor.

The present invention provides use of a 1,4-dihydro-naphthyridine derivative in the treatment of glioma.

The present invention provides use of a 1,4-dihydro-naphthyridine derivative in the treatment of glioblastoma.

The present invention provides use of a 1,4-dihydro-naphthyridine derivative in the treatment of liver cancer, colon cancer and gastric cancer.

The present invention provides use of a 1,4-dihydro-naphthyridine derivative combined with temozolomide in the treatment of glioma.

The present invention provides use of a 1,4-dihydro-naphthyridine derivative combined with radiotherapy in the treatment of glioma.

Advantages and positive effects of the present invention:

As a new drug for treating tumors, the 1,4-dihydro-naphthyridine derivative provided by the present invention is a drug that specifically induces methuosis of tumor cells, which has little impact on normal cells, and is conducive to the development of safe and effective drugs to treat tumors.

## BRIEF DESCRIPTION OF DRAWINGS

Figure 1 shows that 1, 4-dihydro-naphthyridine derivatives induced vacuolation phenotype in T98 cells;

Figure 2 shows that S2 induced vacuolation in various tumor cells;

Figure 3 shows that calcium channel inhibitors did not induce vacuolization of tumor cells;

Figure 4 shows that cholinesterase inhibitors did not induce vacuolation of tumor cells;

Figure 5 shows that calcium channel inhibitors combined with cholinesterase inhibitors did not induce vacuolization of tumor cells;

Figure 6 shows the effect of administration of S2 for 2 weeks on the growth curve of U87MG glioma in the brain of nude mice;

Figure 7 shows that the administration of S2 for 2 weeks inhibited the growth of U87MG glioma in the brain of nude mice;

Figure 8 shows that S2 prolonged the survival period of nude mice with orthotopically transplanted brain glioma;

Figure 9 shows the effect of S2+TMZ combined treatment for 2 weeks on the growth curve of U87MG glioma in the brain of nude mice;

Figure 10 shows the effect of S2+TMZ combined treatment for 2 weeks on the growth of intracerebral glioma;

Figure 11 shows that the S2+TMZ combined treatment prolonged the survival period of mice with U87MG brain carcinoma in situ;

Figure 12 shows that TMZ could not inhibit the growth of T98 glioma and could not reduce the volume and weight of solid tumors;

Figure 13 shows that S2 inhibited the growth of T98 glioma and reduced the volume and weight of solid tumors.

## DETAILED DESCRIPTION

The following examples illustrate the present invention and should not be considered as limiting the present

invention.

**Example 1 Study on the vacuolation-inducing and proliferation-inhibitory effects of 1,4-dihydro-naphthyridine derivatives on glioblastoma cells**

1 Materials and methods

1.1 Cells

Human nerve glioma cell line T98G cells were purchased from Nanjing Cobioer Biotechnology Co., Ltd.

1.2 Number and structure of 1, 4-dihydro-naphthyridine derivatives

| Number of compound | Structure |
|---|---|
| S1 | 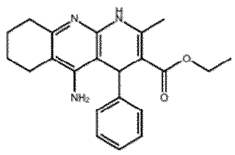 |
| (+)-S2 | 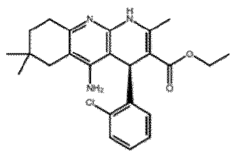 |
| (-)-S2 | 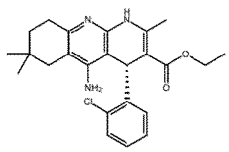 |
| S3 | 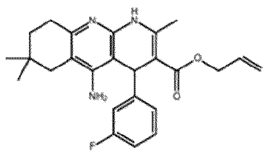 |
| S4 | 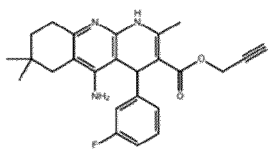 |
| S5 | 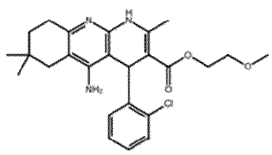 |
| S6 | 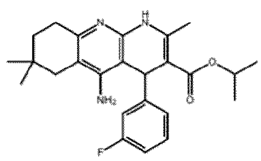 |

1.3 Preparation of compound solution

A certain amount of the compound to be tested was weighed and dissolved with DMSO to prepare a uniform solution with a final concentration of 100 mM.

1.4 Reagents and consumables

| Name | Cat no./Lot no. | Manufacturer |
|---|---|---|
| D-PBS | H106FA0001 | Sangon Biotech |
| Minimum Essential Medium | 2192505 | gibco |
| MEM NEAA(100×) | 2216384 | gibco |
| Sodium Pyruvate(100 mM) | 2192495 | gibco |
| Fetal Bovine Serum | 2261480CP | gibco |
| 0.05% Trypsin-EDTA | 2192791 | gibco |
| DMSO | 21020207 | TEDIA |
| Pen Strep | 2257223 | gibco |
| 12-well plate | 191204-075 | BioFIL |
| 96-well plate | 24820030 | Corning |

1.5 Cell culture and observation of cell phenotype

T98G cells were stably passaged twice. When the cell density reached more than 85%, the cells were digested with 0.05% trypsin-EDTA for 3 minutes, resuspended with the culture medium, and plated in a 12-well plate at a certain density, with each well system being 1 mL. The cells were cultured for 24 hours, and added with the compound solution prepared above to each well according to the grouping, so that the concentrations of the compounds in the culture system were respectively 5 μM, 10 μM, 20 μM, 40 μM, and 60 μM. An equal volume of DMSO was added as a control well.

After 6 hours of drug treatment, the plates were observed under a microscope with a 20× objective lens, and five fields of view were randomly selected for each well to take photos and record. If in the observed field of view, bright vacuoles with clear border appeared in the cell, and at least one of the following conditions was met: 1) a cell contained at least 1 vacuole with a diameter greater than 3 μm; 2) a cell contained 3 or more vacuoles with a diameter within the range of 0.5-3 μm, the cell was considered to be methuosis-positive cells.

$$\text{Vacuolization rate R (\%)} = \text{CELL}_{\text{positive}} / (\text{CELL}_{\text{normal}} + \text{CELL}_{\text{positive}}) \times 100\%$$

Among them, CELL positive: the number of methuosis-positive cells; CELL normal: the number of cells with normal morphology

1.6 Cell proliferation inhibition assay

T98G cells were stably passaged twice. When the cell density reached above 85%, the cells were digested with 0.05% trypsin-EDTA for 3 minutes, resuspended, plated in a 96-well plate at the corresponding density, and cultured for 24 hours. The cells were treated with drugs at final concentrations of the culture system of 0.41 μM, 1.23 μM, 3.7 μM, 11.1 μM, 33.3 μM, and 100 μM, respectively. After 72 hours of drug treatment, cell viability was determined using CellCounting-Lite™ 2.0 kit. The plate was added with reagents at 100 μL/well according to the CellCounting-LiteTM 2.0 kit instructions, and shaken for 10 minutes. The luminescence value (LUM) as read on a multi-function plate reader, and the relative viability of the cells was calculated.

Relative viability of tumor cells V (%) = $(\text{LUM}-\text{LUM}_{\text{background}})/(\text{LUM}_{\text{normal control}} - \text{LUM}_{\text{background}}) \times 100\%$. $\text{LUM}_{\text{background}}$ was the background reading of the complete culture medium well added with the detection reagent. The half inhibitory concentration ($IC_{50}$) was calculated using GraphPad Prism 8.0.1 statistical software.

2 Experimental results

2.1 1, 4-dihydro-naphthyridine derivatives induce vacuolation in T98G cells

S1, S2 (including (+)-S2 and (-)-S2), S3, S4, S5 and S6 can induce vacuolation in T98 (Figure 1 and Table 1), wherein S1 had the weakest vacuolation-inducing activity, and S2 had the strongest vacuolation-inducing activity.

Table 1 Quantitative analysis of vacuolation in T98G cells induced by 1,4-dihydro-naphthyridine derivatives

| | **Vacuolation rate (%)** | | | |
|---|---|---|---|---|
| **Compound** | **5μM** | **10 μM** | **20 μM** | **40 μM** |
| **S1** | 0.0 | 0.3±0.6 | 2.23±0.5 | 10.0±5.6 |
| **(+)-S2** | 1.3±1.6 | 10.0±4.2 | 69.3±6.9 | - |
| **(-)-S2** | 1.2±1.7 | 21.9±9.0 | 96.47±2.4 | - |
| **S3** | 1.1±1.5 | 5.34±1.2 | 9.04±3.8 | 57.4±13.9 |
| **S4** | 0.6±1.4 | 5.0±1.3 | 10.43±1.0 | 76.9±12.1 |
| **S5** | 1.4±1.4 | 10.7±0.90 | 35.1±1.3 | - |
| **S6** | 0.00 | 10.5±1.6 | 15.3±1.3 | 52.3±2.5 |

Data in the table are expressed as mean±SD; "-" indicates that the compound precipitated and the data was not calculated.

2.2 1, 4-dihydro-naphthyridine derivatives inhibit T98G cell proliferation

1, 4-dihydro-naphthyridine derivatives can inhibit the proliferation of neural tumor cells T98G. The tumor proliferation inhibitory activity $IC_{50}$ is shown in Table 2. The proliferation inhibitory activity of S1 is weak, which is basically consistent with the activity of inducing vacuolation.

Table 2 Inhibitory activity of compounds on T98G cell proliferation

| **Compound** | **IC50** |
|---|---|
| S1 | 47.8 μM |
| (+)-S2 | 5.3 μM |
| (-)-S2 | 9.8 μM |
| S3 | 7.0 μM |
| S4 | 12.7 μM |
| S5 | 4.5 μM |
| S6 | 20.5 μM |

Example 2 Study on the inhibitory activity of S2 on tumor cell proliferation *in vitro*

1 Materials and methods

1.1 Cells

Human brain glioma cell line U87MG, human brain glioma cell line T98G and human brain glioma cell line U251 were purchased from Nanjing Cobioer Biotechnology Co., Ltd.; human brain glioma cell line A172, human neuroblastoma cell SK-N-SH, human liver cancer cell line HepG2, mouse colon cancer cell line CT26.WT and hamster ovary cell subline CHO-K1 were purchased from Wuhan Procell Biotechnology Co., Ltd.

1.2 Reagents and consumables

| Name | Cat no./Lot no. | Manufacturer |
|---|---|---|
| D-PBS | H106FA0001 | Sangon Biotech |
| Minimum Essential Medium | 2192505 | gibco |
| MEM NEAA (100×) | 2216384 | gibco |
| Sodium Pyruvate (100 mM) | 2192495 | gibco |
| Fetal Bovine Serum | 2261480CP | gibco |
| 0.05% Trypsin-EDTA | 2192791 | gibco |
| Pen Strep | 2257223 | gibco |
| CellCounting-Lite™ 2.0 | DD1101-02 | Vazyme |
| 96-well plate | 24820030 | Corning |
| 6-well plate | 200421-074B | BioFIL |
| (-)-S2 | D12-20161101-7 | Jiangsu Simovay Pharmaceutical Co., Ltd. |
| DMSO | 21020207 | TEDIA |

1.3 Preparation of drug solution

S2 powder was precisely weighed, and dissolved with DMSO, to prepare a uniform solution with a concentration of 100 mM as the mother solution.

1.4 Cell culture and observation of cell phenotype

U87MG, T98G, U251, SK-N-SH, A-172 and HepG2 cells were adherently cultured in MEM+10%FBS+1%P/S medium and passaged at 1:3; CT26.WT cells were adherently cultured in RPMI- 1640+10% FBS+1% P/S medium and passaged at 1:3; CHO-K1 was cultured adherently in Ham's F-12K+10% FBS+1% P/S medium and passaged at 1:3.

The cells used above were resuscitated and passaged twice stably. When the cell density reached above 85%, the cells were digested with 0.05% trypsin-EDTA for 3 minutes, resuspended with the culture medium, and plated into a 6-well plate at a certain density, with each well system being 2 mL. The cells were cultured for 24 hours, added with 2 μL of the above-prepared S2 solution (final DMSO concentration 0.1%) to each well according to the grouping, such that the S2 concentration in the cell culture system reached 30 μM.

After 6 hours of drug treatment, the plate was observed under a microscope with a 20× objective lens, and five fields of view were randomly selected for each well to take photos and record. If bright vacuoles with clear border appeared in the cell in the observed field of view, and at least one of the following conditions was met: 1) a cell contained at least one vacuole with a diameter greater than 3 μm; 2) a cell contained 3 or more vacuoles with a diameter within the range of 0.5-3 μm, the cell was considered as a methuosis-positive cell (Journal of Medicinal Chemistry 2018 61 (12), 5424-5434).

$$\text{Vacuolization rate R (\%)} = \text{CELL}_{\text{positive}} / (\text{CELL}_{\text{normal}} + \text{CELL}_{\text{positive}}) \times 100\%$$

CELL positive: the number of methuosis-positive cells; $\text{CELL}_{\text{normal}}$: the number of cells with normal morphology

1.5 Cell viability assay

The above various cells were resuscitated and stably passaged twice. When the cell density reached above 85%, the cells were digested with 0.05% trypsin-EDTA for three minutes, resuspended, plated in a 96-well plate at the corresponding density, and cultured for 24 hours. The cells were treated with drugs at final concentrations of the culture system of 0.41 μM, 1.23 μM, 3.7 μM, 11.1 μM, 33.3 μM, and 100 μM. After 48 hours of drug treatment, cell viability was determined using CellCounting-Lite™ 2.0 kit. The plate was added with reagents at 100 μL/well according to the CellCounting-LiteTM 2.0 kit instructions, and shaken for 10 minutes. The luminescence value (LUM) was read on a multi-function plate reader, and the relative viability of the cells was calculated.

Relative viability of tumor cells V (%) = $(\text{LUM}-\text{LUM}_{\text{background}})/(\text{LUM}_{\text{normal control}} - \text{LUM}_{\text{background}}) \times 100\%$. $\text{LUM}_{\text{background}}$ was the background reading of the complete culture medium well added with the detection reagent. The half inhibitory concentration ($IC_{50}$) was calculated using GraphPad Prism 8.0.1 statistical software.

2 Experimental results

2.1 S2 induces vacuolization of tumor cells

S2 can induce vacuolation in neural tumor cells such as human glioma cell lines U87MG, T98G, U251 and A172, human neuroblastoma cell line SK-N-SH (Figure 2A-D), and can also induce vacuolation in non-nervous system tumor cells (Figure 2 A-D), including human liver cancer cell HepG2 and mouse colon cancer cell CT26.WT. However, S2 could not induce vacuolation in the non-tumor cell line hamster ovary cell CHO-K1.

2.2 S2 inhibits tumor cell proliferation

S2 can inhibit the proliferation of neural tumor cells and non-neural tumor systems, with an $IC_{50}$ within the range of 4.7-14.3 μM, but had very weak inhibitory effect on the proliferation of non-tumor cell lines ($IC_{50}$> 100 μM) (Table 3).

Table 3 Inhibitory effect of S2 on tumor cell proliferation

| Tumor cell | $IC_{50}$ (μM) |
|---|---|
| U87MG | 11.7 μM |
| T98G | 9.4 μM |
| U251 | 14.3 μM |
| SK-N-SH | 11.4 μM |
| A172 | 4.7 μM |
| HepG2 | 10.1 μM |
| CT26.WT | 7.1 μM |
| CHO-K1 | ~100 μM |

Example 3 Effects of AChE inhibitors and VGCC blockers on the vacuolation phenotype of T98 cells

1 Materials and methods

1.1 Cells

Human brain glioma cell line T98G was purchased from Nanjing Cobioer Biotechnology Co., Ltd.

1.2 Reagents and consumables

| Name | Cat no./Lot no. | Manufacturer |
|---|---|---|
| D-PBS | H106FA0001 | Sangon Biotech |
| Minimum Essential Medium | 2192505 | gibco |
| MEM NEAA (100×) | 2216384 | gibco |
| Sodium Pyruvate (100 mM) | 2192495 | gibco |
| Fetal Bovine Serum | 2261480CP | gibco |
| 0.05% Trypsin-EDTA | 2192791 | gibco |
| Pen Strep | 2257223 | gibco |
| 12-well plate | 191204-075 | BioFIL |
| (-)-S2 | D12-20161101-7 | Jiangsu Simovay Pharmaceutical Co., Ltd. |
| Nifedipine | N7634 | Sigma |
| Diltiazem | 20210815 | Nanjing Chemlin Chemical Industry Co.,Ltd. |
| Verapamil | C13156635 | Shanghai Macklin Biochemical Technology Co., Ltd. |

(continued)

| Name | Cat no./Lot no. | Manufacturer |
|---|---|---|
| Donepezil | DT6821 | Sigma |
| Flunarizine | AGN20-39525 | Shanghai Kaiwei Chemical Technology Co., Ltd. |
| DMSO | 21020207 | TEDIA |

1.3 Preparation of drug solution

The drugs to be tested include: nifedipine, verapamil, diltiazem, flunarizine and donepezil.

The above-mentioned drugs to be tested were precisely weighed, and dissolved with DMSO, to prepare a uniform solution with a concentration of 20 mM as the mother solution, respectively.

1.4 Cell culture and observation of cell phenotype

T98G cells were stably passaged twice. When the cell density reached more than 85%, the cells were digested with 0.05% trypsin-EDTA for 3 minutes, resuspended with the culture medium, and plated in a 12-well plate at a certain density, with each well system being 1 mL. The cells were cultured for 24 hours, and added with the compound solution prepared above to each well according to the grouping, such that the compound concentrations in the culture system were 0.1 μM, 1 μM, 5 μM, and 20 μM, respectively. An equal volume of DMSO was added as a control well.

After 6 hours of drug treatment, the plate was observed under a microscope with a 20× objective lens. If bright vacuoles with clear border appeared in the observed field of view, and at least one of the following conditions was met: 1) a cell contained at least one vacuole with a diameter greater than 3 μm; 2) a cell contained 3 or more vacuoles with a diameter within the range of 0.5-3 μm; the cell was considered as a methuosis-positive cell.

2 Experimental results

2.1 Calcium channel inhibitors (VGCC) failed to induce vacuolization of tumor cells

None of calcium channel inhibitors nifedipine, verapamil, diltiazem, and flunarizine with a maximum drug concentration of 20 μM could induce a vacuolated cell phenotype of the human glioma cell line T98G (Figure 3).

2.2 Cholinesterase (AChE) inhibitors failed to induce vacuolization of tumor cells

The cholinesterase inhibitor donepezil with a maximum drug concentration of 20 μM failed to induce the vacuolated cell phenotype of the human glioma cell line T98G (Figure 4).

2.3 Calcium channel inhibitors in combination with cholinesterase inhibitors failed to induce vacuolization of tumor cells

The combined use of the calcium channel inhibitor nifedipine or verapamil (20 μM) with the cholinesterase inhibitor donepezil (20 μM) failed to induce the vacuolated cell phenotype in the human glioma cell line T98G (Figure. 5).

Example 4 Study on the efficacy of S2 on U87MG-Luc glioblastoma cell in situ transplantation tumor model in nude mice

1 Materials and methods

1.1 Experimental animals

BALB/c nude mice, male, SPF-grade, weighing 20-22 g, were purchased from Shanghai Lingchang Biotechnology Co., Ltd.

1.2 Reagents and consumables

The information of the main reagents used in this experiment is as follows:

| Name | Cat no./Lot no. | Manufacturer |
|---|---|---|
| D-PBS | H106FA0001 | Sangon Biotech |
| Minimum Essential Medium | 2192505 | gibco |
| MEM NEAA(100×) | 2216384 | gibco |
| Sodium Pyruvate(100 mM) | 2192495 | gibco |
| Fetal Bovine Serum | 2261480CP | gibco |
| 0.05% Trypsin-EDTA | 2192791 | gibco |
| DMSO | 21020207 | TEDIA |
| Double antibodies | 2257223 | gibco |
| 0.9% sodium chloride injection | 1903224C | Anhui Double-Crane Pharmaceutical Co., Ltd. |
| (-)-S2 | D12-20161101-7 | Jiangsu Simovay Pharmaceutical Co., Ltd. |
| Temozolomide (TMZ) | B1828132 | Shanghai Aladdin Biochemical Technology Co., Ltd. |
| Solutol HS-15 (Kolliphor HS-15) | 09362556P0 | Shanghai Chineway Pharmaceutical Excipients Technology Co., Ltd. |
| Ethanol | 20180419 | Sinopharm Chemical Reagent Co., Ltd. |
| Glucose | 20161219 | Sinopharm Chemical Reagent Co., Ltd. |
| Isoflurane | 21101001 | Ruiwode Life Technologies Co., Ltd. |

1.3 Experimental equipment

The main equipment information in this experiment is as follows:

| Equipment name | Manufacturer | Model | Device number |
|---|---|---|---|
| Analytical Balances | Mettler toledo | XA105 | OW020004 |
| Electronic scale | Changshu G&G Measurement Plant | T1000 type | N/A |
| Small animal five imager | PE | IVIS Lumina XR | N/A |
| Stereotaxic instrument | Stoelting | / | N/A |
| Disposable sterile syringe | Shanghai Misawa Medical Industry Co.,Ltd. | 1 mL | N/A |

1.4 Preparation of drug solutions

S2

Solvent: 5% ethanol + 0.8% Solutol + 5% glucose

Preparation method: An appropriate amount of S2 was taken, dissolved with absolute ethanol in proportion under ultrasound, mixed evenly with Solutol HS-15 in proportion under ultrasound (no filamentation phenomenon was observed), slowly added with purified water for dissolution during the ultrasound process, added with glucose in proportion, and dissolved under ultrasound to obtain a solution. The solution was stored at 4°C in the dark.

1.5 Experimental methods

1.5.1 Establishment of U87MG-Luc glioblastoma cell in situ transplantation tumor model in nude mice

Before the transplantation, the U87MG-Luc cells were digested with 0.05%-trypsin-EDTA, resuspended with pre-cooled PBS to $4\times10^7$ cells/mL, and transferred to ice for later use. The nude mice were anesthetized with isoflurane gas, and then fixed on a stereotaxic machine in a prone position. The scalp of the nude mouse was disinfected with iodophor, and incised sagittally with a scalpel. The incision was cleaned with iodophor. The skull was exposed, and drilled with a skull drill extending 1.0 mm in front of the bregma and deviating the right side 2.0 mm. 5 μL of cell suspension ($2\times10^5$ cells) was manually injected with a 10 μL flat-head microsyringe, with inserting the needle to a depth of 3.5 mm,

withdrawing the needle 0.5 mm, and injecting for about 10 minutes. After stopping the injection for 5 minutes, the needle was slowly removed, and the incision was sterilized and sutured. Finally 50,000 units of penicillin was injected intramuscularly to prevent infection.

1.5.2 Animal grouping and administration

This experiment was divided into 5 groups, namely model group, TMZ group, S2 administration group-LD (0.25 mg/kg), S2 administration group-MD (0.5 mg/kg), S2 administration group-HD (1 mg/kg). The modeled animals were divided into each group in a single-blind manner with equal probability. From the 5th day after the transplantation, the drugs were administered intravenously once a day for 15 days. The model group was given solvent intravenously once a day for 15 days.

1.5.3 *In vivo* imaging of tumor cells in the brain

On days 4, 12, 18, and 25 after the transplantation, 200 μL of 15 mg/mL D-luciferin potassium salt solution, which was filtered and sterilized through a 0.22 μm filter, was injected intraperitoneally. After 10 minutes, IVI chemiluminescence detection (bright field + bioluminescence imaging) was performed using the mouse *in vivo* imaging system (PE company IVIS Lumina XR). Luorescence intensity was analyzed using Living Image software to obtain fluorescence intensity [p/s], which reflected the size of tumor tissue in the brain of experimental animals.

1.5.4 Animal survival period records

The survival conditions of experimental animals were monitored, and the animals' death dates were recorded timely.

1.6 Data statistical analysis

Quantitative data are expressed as mean ± standard error. Each pharmacodynamic index was plotted using GraphPad Prism (8.0.1) software, differences between groups were tested by one-way ANOVA or two-way repeat ANOVA, followed by Fisher's LSD test, and survival analysis was performed by Kaplan-Meier. $P < 0.05$ was defined as a significant difference.

2 Experimental results

2.1 S2 inhibited the growth of U87MG glioma transplanted in situ in the brain of nude mice

Compared with the vehicle group, both the S2 administration group-MD (0. 5 mg/kg, IV) and the S2 administration group-HD (1 mg/kg, IV) could significantly inhibit the growth of U87MG in situ tumors in the brain (Figure 6 A and B). After two weeks of administration, S2 administration group-MD (0.5 mg/kg, IV) and S2 administration group-HD (1 mg/kg, IV) showed significant inhibitory effect on tumor growth compared with the vehicle group, wherein the S2 administration group-HD (1 mg/kg, IV) had the strongest inhibitory effect on tumor (Figure 7 A and B).

2.2 S2 prolonged the survival period of nude mice with glioma transplanted in situ in the brain

According to the survival curve Log-rank analysis, compared with the vehicle group, S2 administration group-MD (0.5 mg/kg, IV) and S2 administration group-HD (1 mg/kg, IV) significantly increased the survival rate of mice with U87MG brain carcinoma in situ (Figure 8), prolonged median survival period and overall survival period (Table 4).

Table 4 S2 prolonged the survival period of mice with U87MG brain carcinoma in situ

| Survival period | **Vehicle** | **S2 0.25 mg/kg** | **S2 0.5 mg/kg** | **S2 1 mg/kg** |
|---|---|---|---|---|
| Median survival period **(days)** | 31.5 | 32.5 | 33.5 | 35 |
| Overall survival period **(days)** | 31 | 32 | 33 | 36 |

Example 5 Study on the efficacy of S2 in combination with temozolomide (TMZ) on the U87MG-Luc glioblastoma cell in situ transplantation tumor model in nude mice

1 Materials and methods

1.1 Experimental animals

Same as Example 4.

1.2 Reagents and consumables

Same as Example 4.

1.3 Experimental equipment

Same as Example 4.

1.4 Preparation of drug solutions

a) S2

Solvent: 5% ethanol + 0.8% Solutol + 5% glucose

Preparation method: An appropriate amount of S2 was taken, dissolved with absolute ethanol in proportion under ultrasound, mixed evenly with Solutol HS-15 in proportion under ultrasound (no filamentation phenomenon was observed), slowly added with purified water for dissolution during the ultrasound process, added with glucose in proportion, and dissolved under ultrasound to obtain a solution. The solution was stored at 4°C in the dark.

b) TMZ

Solvent: 0.9% sodium chloride injection

Preparation method: An appropriate amount of TMZ was weighed, diluted with 0.9% sodium chloride injection in proportion, and shaken until evenly mixed to obtain a solution. The solution was stored at -20°C.

1.5 Experimental methods

1.5.1 Establishment of U87MG-Luc glioblastoma cell in situ transplantation tumor model in nude mice

Same as Example 4.

1.5.2 Animal grouping and administration

In this experiment, the mice were divided into 4 groups, namely model group, TMZ (3 mg/kg) group, S2+TMZ combined administration group-LD (TMZ 3 mg/kg and S2 0.25 mg/kg), S2+TMZ combined administration group-MD (TMZ 3 mg/kg and S2 0.5 mg/kg) and S2+TMZ combined administration group-HD (TMZ 3 mg/kg and S2 1 mg/kg). The modeled animals were divided into each group in a single-blind manner with equal probability. From the 8th day after the transplantation, TMZ was intraperitoneally administered twice on the 8th and 15th days; S2 was intravenously administered once a day for 15 days.

1.5.3 *In vivo* imaging of tumor cells in the brain

On days 7, 14, and 21 after the transplantation, 200 μL of 15 mg/mL D-luciferin potassium salt solution, which was filtered and sterilized through a 0.22 μm filter, was injected intraperitoneally. After 10 minutes, IVI chemiluminescence detection (bright field + bioluminescence imaging) was performed using the mouse *in vivo* imaging system (PE company IVIS Lumina XR). Luorescence intensity was analyzed using Living Image software to obtain fluorescence intensity [p/s], which reflected the size of tumor tissue in the brain of experimental animals.

1.5.4 Animal survival records

The survival conditions of experimental animals were monitored, and the animals' death dates were recorded

timely.

1.6 Data statistical analysis

Quantitative data are expressed as mean ± standard error. Each pharmacodynamic index was plotted using GraphPad Prism (8.0.1) software, differences between groups were tested by one-way ANOVA or two-way repeat ANOVA, followed by Fisher's LSD test, and survival analysis was performed by Kaplan-Meier. $P < 0.05$ was defined as a significant difference.

2 Experimental results

2.1 Effect of S2+TMZ combination on the growth of U87MG glioma in the brain of nude mice

Compared with the vehicle group, TMZ (3 mg/kg, IP) administration alone and TMZ+S2 (0.25, 0.5, and 1 mg/kg IV) combined administration could both significantly inhibit the growth of U87MG in situ tumor in the brain (Figure 9 A and B). After 2 weeks of administration (day 21), compared with TMZ, the S2 + TMZ combined administration group showed stronger inhibitory effect on tumor growth, and the tumor inhibitory effect of the S2 (0.5 mg/kg) + TMZ combined administration group was significantly better than that of the TMZ group (Figure. 10 A and B).

2.2 Effect of S2+TMZ combination on survival of nude mice with in situ glioma in brain

According to the survival curve Log-rank analysis, compared with the vehicle group, there was a significant difference in the survival period of mice between the TMZ (3 mg/kg, IP) alone group and TMZ+S2 (0.25, 0.5 and 1 mg/kg IV) combined administration group. Compared with TMZ administration alone, the combination of S2 (0.5 mg/kg) + TMZ and S2 (1 mg/kg) + TMZ significantly increased the survival rate of mice with U87MG brain carcinoma in situ (Figure 11) and prolonged the median survival period and overall survival period (Table 5).

Table 5 The combined use of S2+TMZ prolonged the survival period of mice with U87MG brain carcinoma in situ.

| **Survival period** | **Vehicle** | **TMZ 3 mg/kg** | **TMZ+ S2 0.25 mg/kg** | **TMZ+ S2 0.5 mg/kg** | **TMZ+ S2 1 mg/kg** |
|---|---|---|---|---|---|
| Median survival period **(days)** | 30 | 33.5 | 32 | 35 | 36 |
| Overall survival period **(days)** | 33 | 35 | 36 | 37 | 38 |

Example 6 *In vivo* pharmacodynamic study of S2 on human brain glioma T98G in NOD SCID mouse subcutaneous model

1 Methods and materials

1.1 Experimental animals

NOD SCID, female, 6-8-week old, weighing 18-21 g, were purchased from Beijing VitalRiver Experimental Animal Technology Co., Ltd.

1.2 Tested drugs

Temozolomide (TMZ), Shanghai Aladdin Biochemical Technology Co., Ltd., batch number: B1828132.

S2, Jiangsu Simovay Pharmaceutical Co., Ltd., batch number: D12-20161101-7.

1.3 Preparation of drug solutions

Preparation method of TMZ (1.5 mg/mL): 18 mg of Temozolomide was weighed and dissolved in 12 mL of 1% HPMC.

The preparation method of S2 administration solution was the same as that in Example 4.

1.4 Experimental methods

1.4.1 Establishment of T98G glioblastoma cells subcutaneous transplantation tumor model in nude mice

Human brain glioma T98G cells were cultured *in vitro*. The cells were cultured in EMEM medium added with 10% fetal calf serum, 100 U/mL penicillin and 100 μg/mL streptomycin, in a 37°C 5% $CO_2$ incubator. After the number of cells reached the required number, the cells were collected. 0.2 mL ($10 \times 10^6$) of T98G tumor cells (PBS: Matrigel = 1:1) were subcutaneously inoculated into the right back of each mouse. The mice were grouped and administered when the average tumor volume reached approximately 147 $mm^3$.

1.4.2 Animal grouping and administration

The first set of experiments was divided into two groups, namely: vehicle group and TMZ group (15 mg/kg, PO); the modeled animals were randomly divided into each group in a single-blind manner, with 10 animals in each group.

The second set of experiments was divided into two groups, namely: vehicle group and S2 group (3 mg/kg, IV); the modeled animals were randomly divided into each group in a single-blind manner, with 9 animals in each group.

1.4.3 Measurement of tumor size

Tumor diameters were measured with vernier calipers twice a week, and tumors were weighed and photographed after the experiment.

1.5 Data Statistics The tumor volume was calculated as follows: $V = 0.5a \times b^2$, where a and b represent the long and short diameters of the tumor respectively.

The tumor inhibitory effect was evaluated by TGI (%) or relative tumor proliferation rate T/C (%).

The tumor inhibitory effect was calculated as TGI (%): TGI % = [1-(average tumor volume at the end of administration in a certain treatment group - average tumor volume at the beginning of administration in the treatment group) / (average tumor volume at the end of administration in the vehicle control group - average tumor volume in the vehicle control group at the beginning of treatment)] × 100.

Relative tumor proliferation rate T/C (%): T/C % = TRTV / CRTV × 100 (TRTV: RTV of treatment group; CRTV: RTV of negative control group). The relative tumor volume (RTV) was calculated based on the results of tumor measurement according to the calculation formula of $RTV = V_t / V_0$, where $V_0$ is the average tumor volume measured at the time of grouping and administration (i.e. $d_0$), and $V_t$ is the average tumor volume of the measurement at a certain time, $T_{RTV}$ and $C_{RTV}$ are taken on the same day.

Statistical analysis was based on the mean and standard error (SEM) of tumor volumes in each group at the end of the experiment. Comparisons between two groups were analyzed using T-test. All data analysis was performed with GraphPad Prism (8.0.1), and $P < 0.05$ was considered to be a significant difference.

2 Experimental results

2.1 Effect of TMZ on T98 tumor growth and tumor weight at experimental endpoint

Compared with the vehicle group, the TMZ 15 mg/kg group had no inhibitory effect on tumor volume growth (Figure 12 A-C). Tumors were collected and weighed at the end of the experiment. There was no significant difference in tumor weight between the TMZ 15 mg/kg group and the control group (Figure 12 D), which was consistent with the above tumor volume inhibition.

2.2 Effect of S2 on T98 tumor growth and tumor weight at experimental endpoint

Compared with the vehicle group, the S2 3 mg/kg group significantly inhibited tumor volume growth (Figure 13 A-C). Tumors were collected and weighed at the end of the experiment. The tumor weight in the S2 3 mg/kg group was significantly lower than that in the control group (Figure 13 D), which is consistent with the above-mentioned tumor volume inhibition.

## Claims

1. Use of a 1,4-dihydro-naphthyridine derivative represented by Formula I or a pharmaceutically acceptable salt thereof in the manufacture of a medicament for treating a tumor,

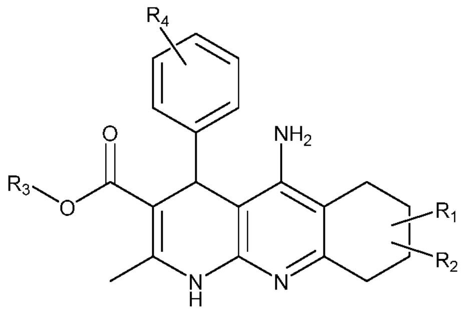

Formula I

wherein, $R_1$ or $R_2$ is hydrogen, halogen or $C_1$-$C_6$ alkyl;
$R_3$ is selected from $C_1$-$C_6$ alkyl, wherein any $-CH_2-$ in the alkyl may be replaced by one or more -O-;
$R_4$ is hydrogen or halogen.

**2.** Use of a 1,4-dihydro-naphthyridine derivative or a pharmaceutically acceptable salt thereof in the manufacture of a medicament for treating a tumor, wherein the compound is selected from the group consisting of:

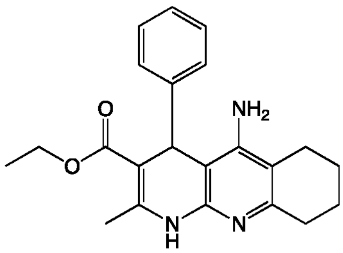

**S1**

Compound 1 represented by S1;

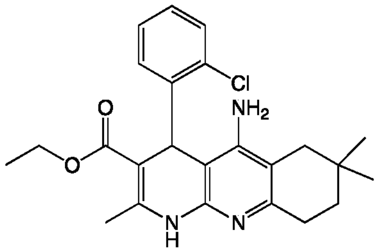

**S2**

Compound 2 represented by S2;

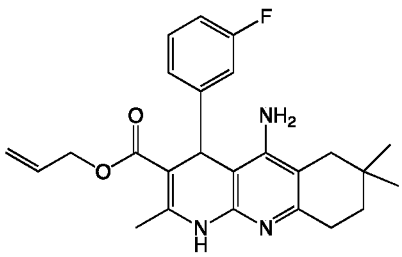

**S3**

Compound 3 represented by S3;

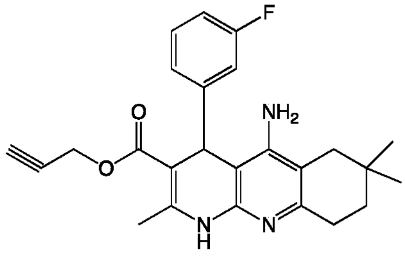

S4

Compound 4 represented by S4;

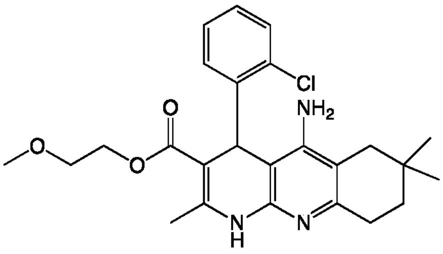

S5

Compound 5 represented by S5; and

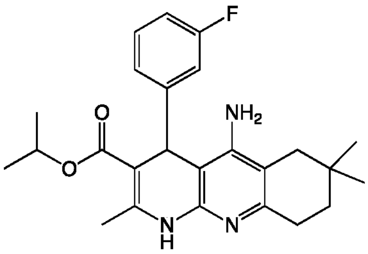

S6

Compound 6 represented by S6.

**3.** The use according to claim 2, wherein the 1,4-dihydro-naphthyridine derivative is used for treating a solid tumor.

**4.** The use according to claim 2, wherein the 1,4-dihydro-naphthyridine derivative is used for treating a nervous system tumor.

**5.** The use according to claim 2, wherein the 1,4-dihydro-naphthyridine derivative is used for treating a brain tumor.

**6.** The use according to claim 2, wherein the 1,4-dihydro-naphthyridine derivative is used for treating a glioma.

**7.** The use according to claim 2, wherein the 1,4-dihydro-naphthyridine derivative is used for treating a glioblastoma.

**8.** The use according to claim 2, wherein the 1,4-dihydro-naphthyridine derivative is used for treating liver cancer, colon cancer and gastric cancer.

**9.** The use according to claim 2, wherein the 1,4-dihydro-naphthyridine derivative is used in combination with temozolomide for treating a glioma.

**10.** The use according to claim 2, wherein the 1,4-dihydro-naphthyridine derivative is used in combination with radiotherapy for treating a glioma.

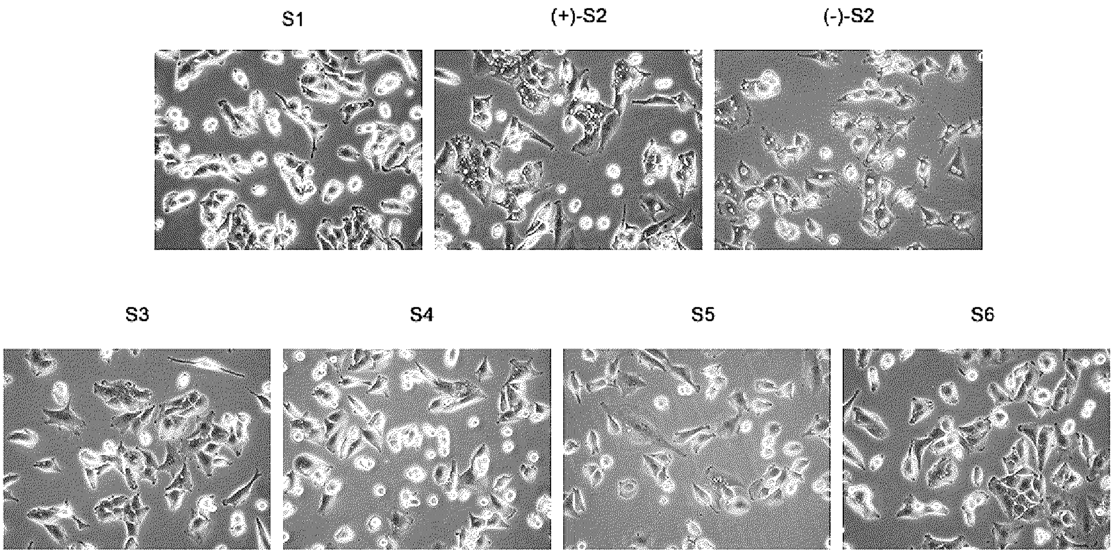


FIG.1

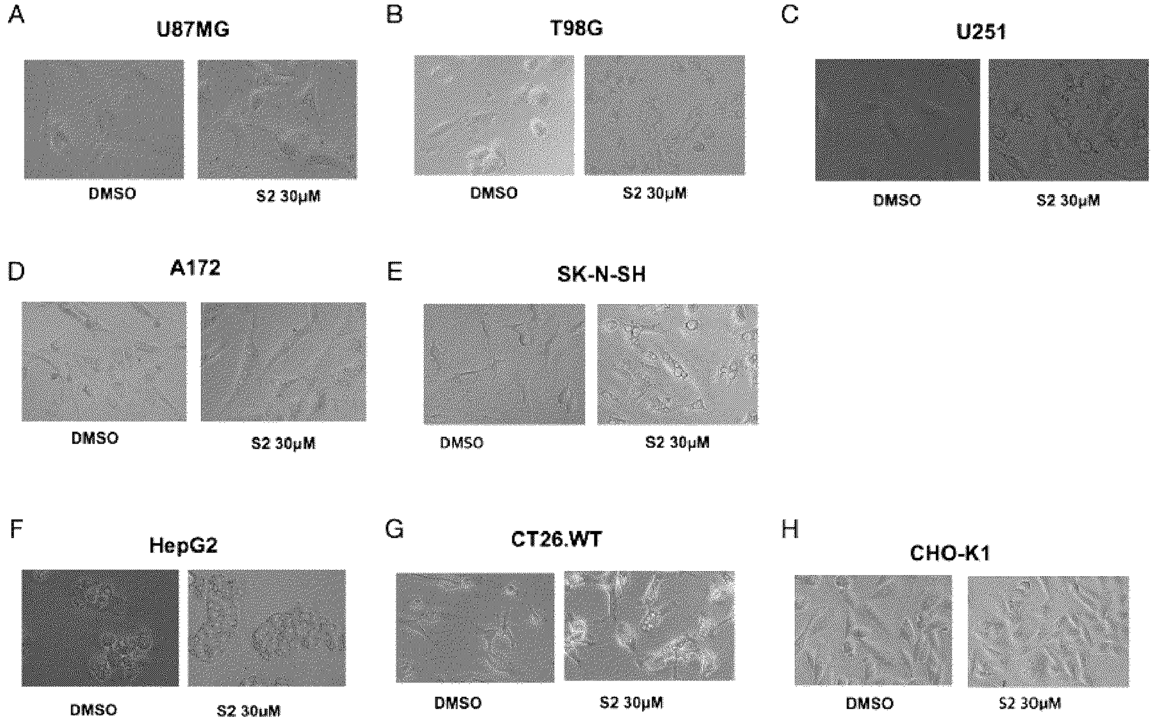
A
U87MG
DMSO
S2 30μM
B
T98G
DMSO
S2 30μM
C
U251
DMSO
S2 30μM
D
A172
DMSO
S2 30μM
E
SK-N-SH
DMSO
S2 30μM
F
HepG2
DMSO
S2 30uM
G
CT26.WT
DMSO
S2 30μM
H
CHO-K1
DMSO
S2 30μM

FIG.2

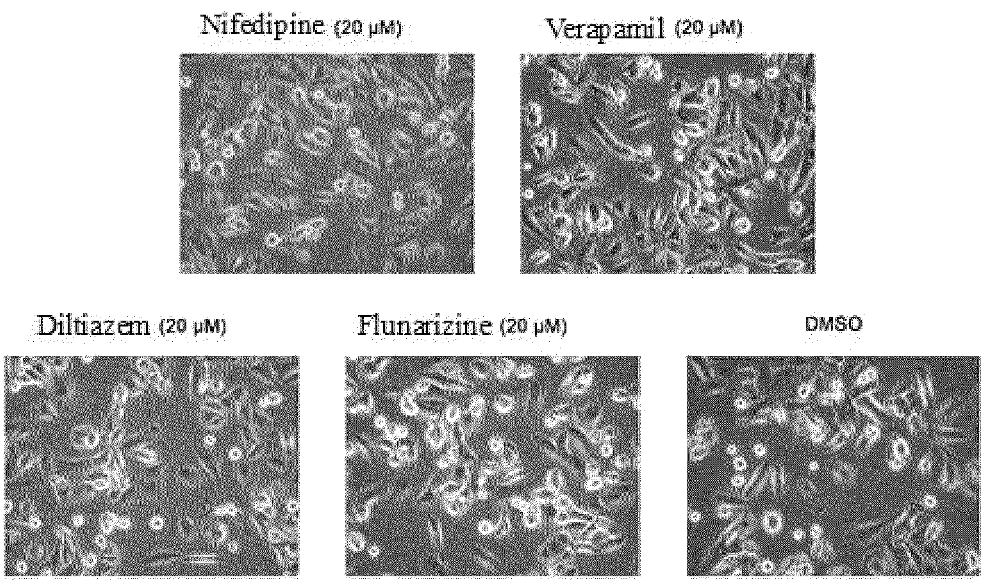
Nifedipine (20 μM)
Verapamil (20 μM)
Diltiazem (20 μM)
Flunarizine (20 μM)
DMSO

FIG.3

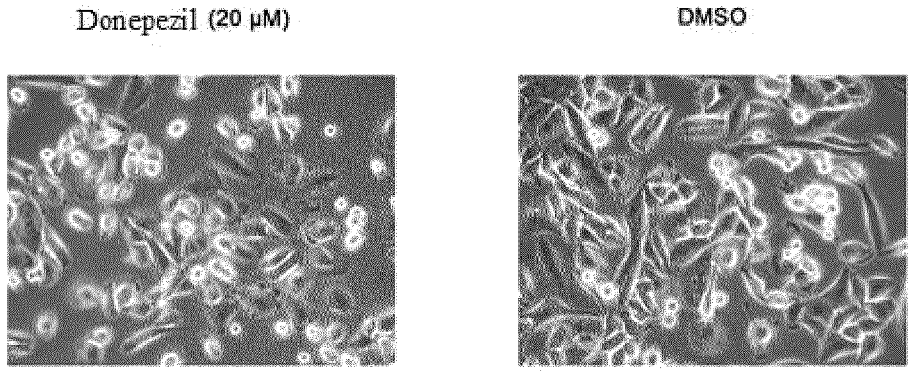
Donepezil (20 µM)
DMSO

FIG.4

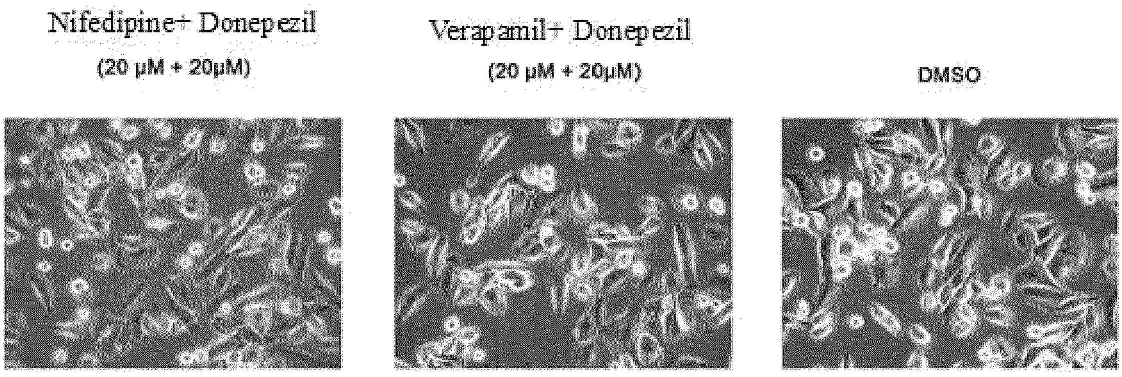
Nifedipine+ Donepezil
(20 µM + 20µM)
Verapamil+ Donepezil
(20 µM + 20µM)
DMSO

FIG.5

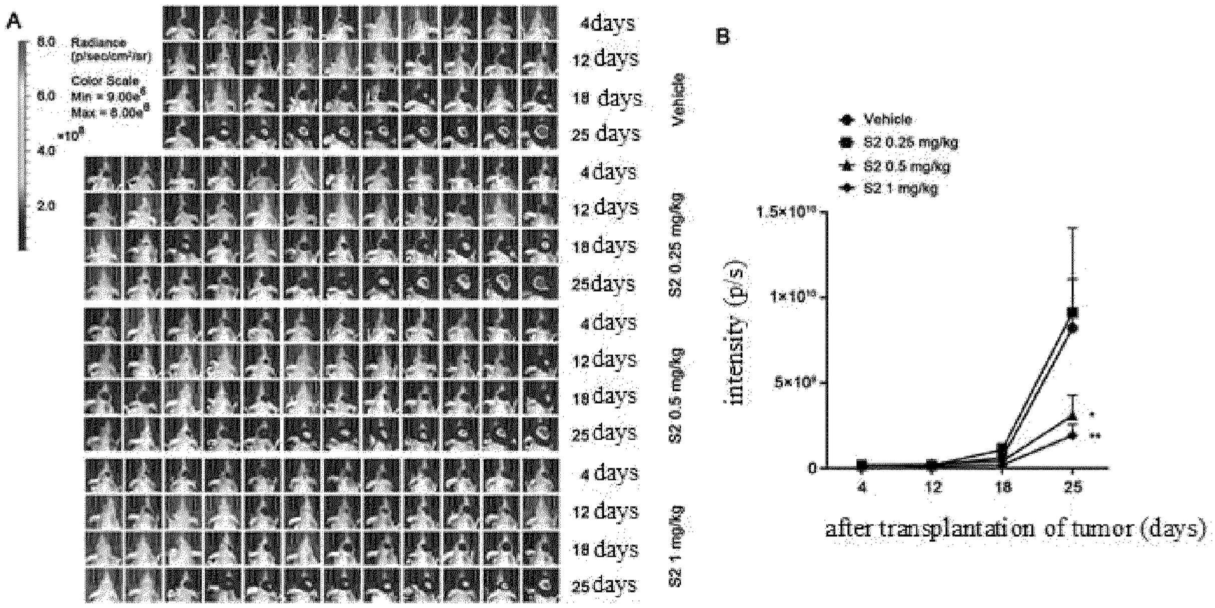
A
Radiance (p/sec/cm²/sr)
Color Scale Min = 9.00e6 Max = 8.00e8
4days
12 days
18 days
25 days
Vehicle
S2 0.25 mg/kg
S2 0.5 mg/kg
S2 1 mg/kg
B
Vehicle
S2 0.25 mg/kg
S2 0.5 mg/kg
S2 1 mg/kg
intensity (p/s)
after transplantation of tumor (days)

FIG.6

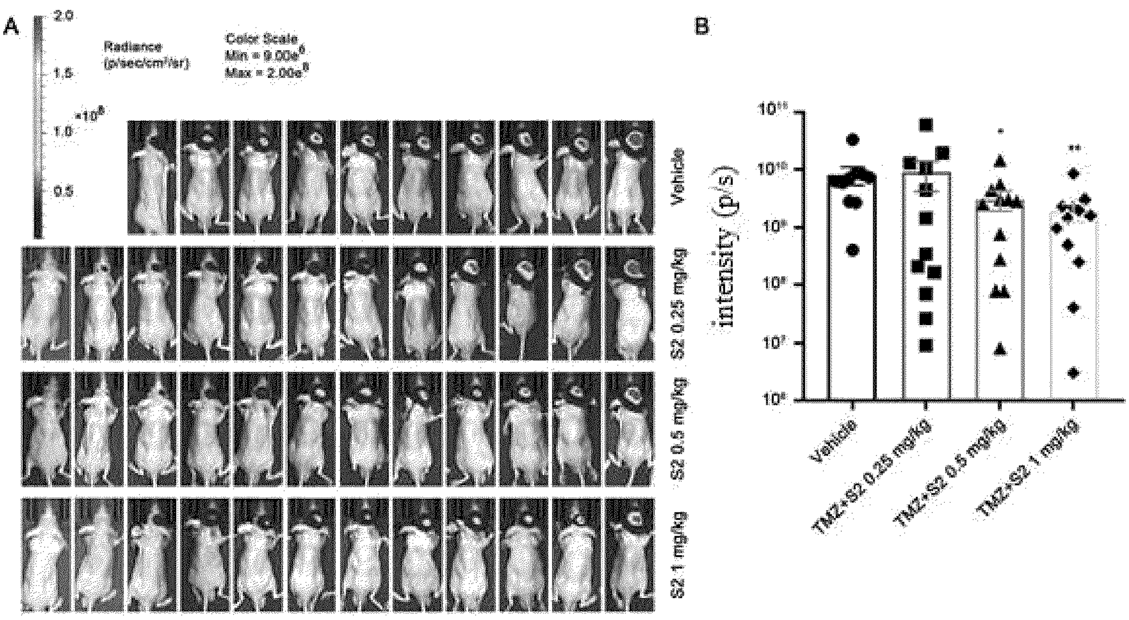
A
Radiance (p/sec/cm²/sr)
Color Scale Min = 9.00e6 Max = 2.00e8
Vehicle
S2 0.25 mg/kg
S2 0.5 mg/kg
S2 1 mg/kg
B
intensity (p/s)
Vehicle
TMZ+S2 0.25 mg/kg
TMZ+S2 0.5 mg/kg
TMZ+S2 1 mg/kg

FIG.7

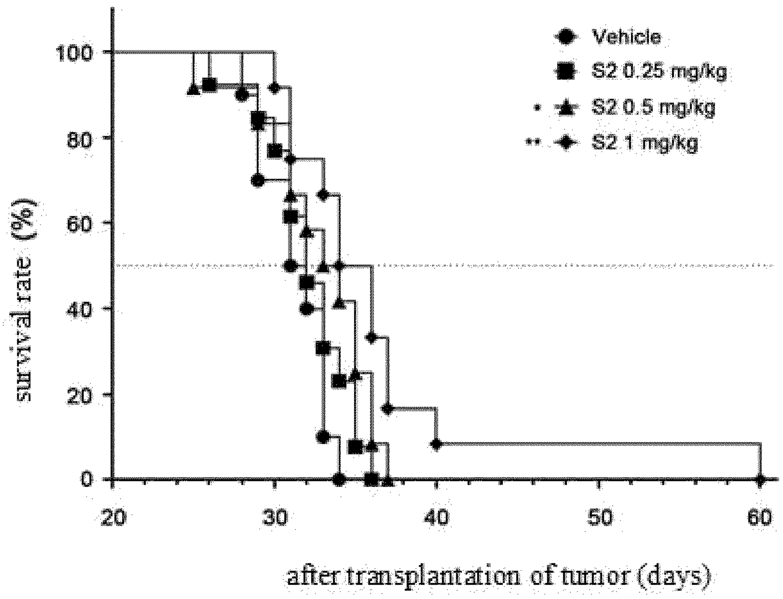
Vehicle
S2 0.25 mg/kg
* S2 0.5 mg/kg
** S2 1 mg/kg
survival rate (%)
after transplantation of tumor (days)

FIG.8

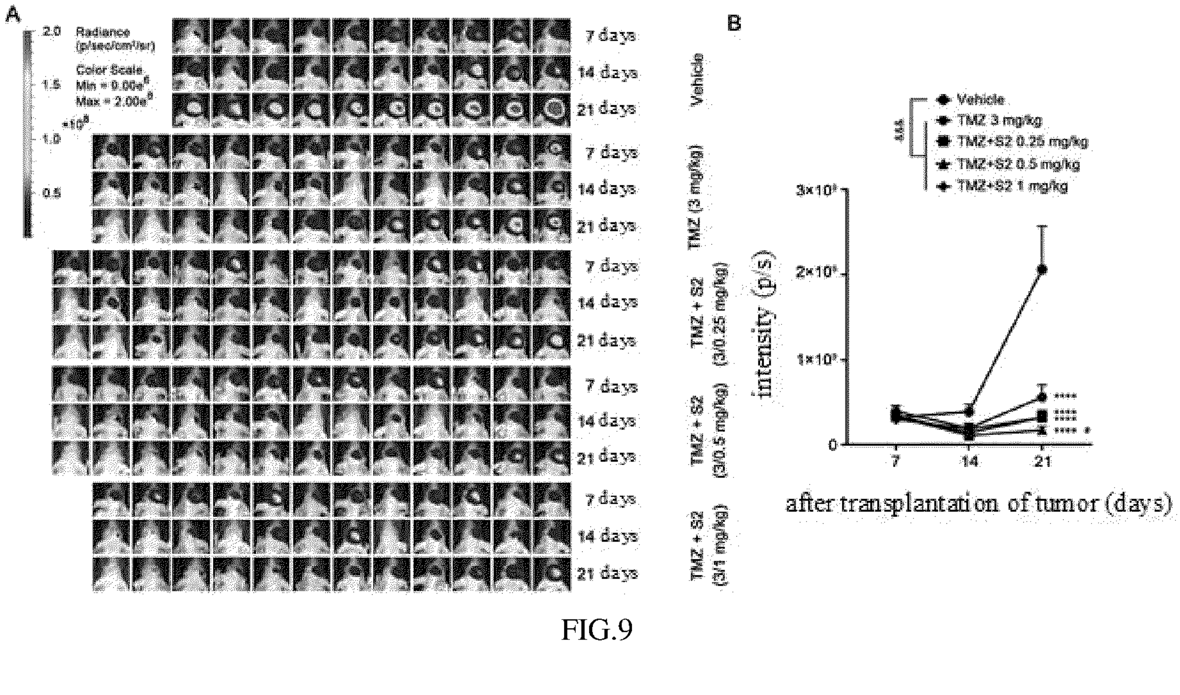
A
B
7 days
14 days
21 days
Vehicle
TMZ (3 mg/kg)
TMZ + S2 (3/0.25 mg/kg)
TMZ + S2 (3/0.5 mg/kg)
TMZ + S2 (3/1 mg/kg)
Vehicle
TMZ 3 mg/kg
TMZ+S2 0.25 mg/kg
TMZ+S2 0.5 mg/kg
TMZ+S2 1 mg/kg
intensity (p/s)
after transplantation of tumor (days)

FIG.9

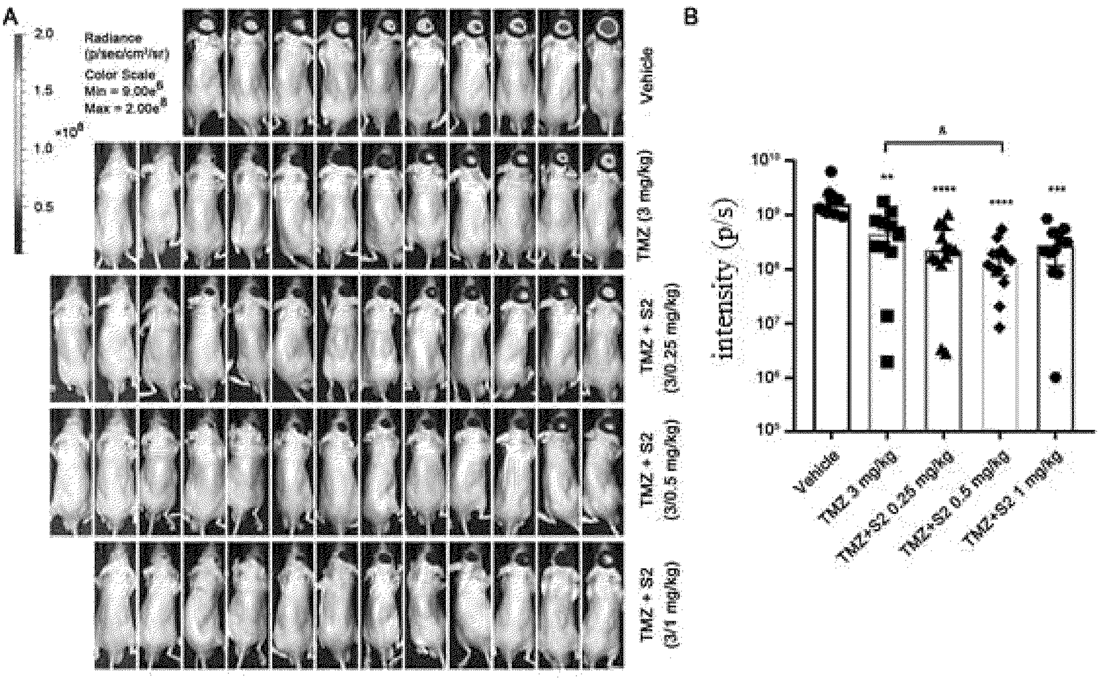
A
Radiance
(p/sec/cm²/sr)
Color Scale
Min = 9.00e8
Max = 2.00e8
×10⁸
2.0
1.5
1.0
0.5
Vehicle
TMZ (3 mg/kg)
TMZ + S2 (3/0.25 mg/kg)
TMZ + S2 (3/0.5 mg/kg)
TMZ + S2 (3/1 mg/kg)
B
intensity (p/s)
Vehicle
TMZ 3 mg/kg
TMZ+S2 0.25 mg/kg
TMZ+S2 0.5 mg/kg
TMZ+S2 1 mg/kg

FIG.10

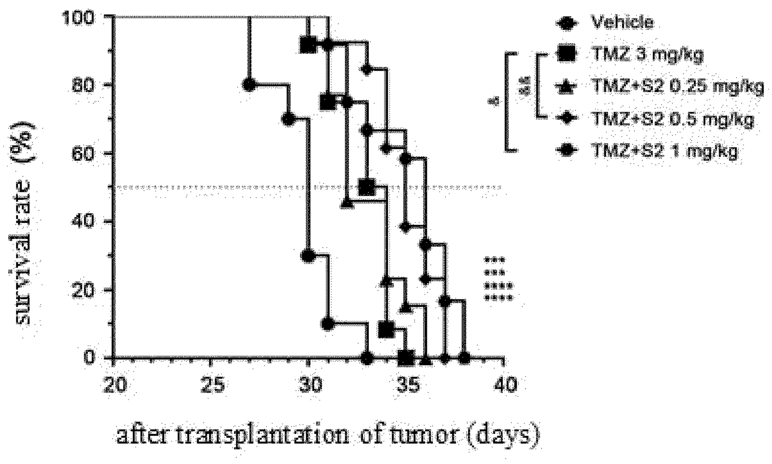
Vehicle
TMZ 3 mg/kg
TMZ+S2 0.25 mg/kg
TMZ+S2 0.5 mg/kg
TMZ+S2 1 mg/kg
survival rate (%)
after transplantation of tumor (days)
100
80
60
40
20
0
20
25
30
35
40

FIG.11

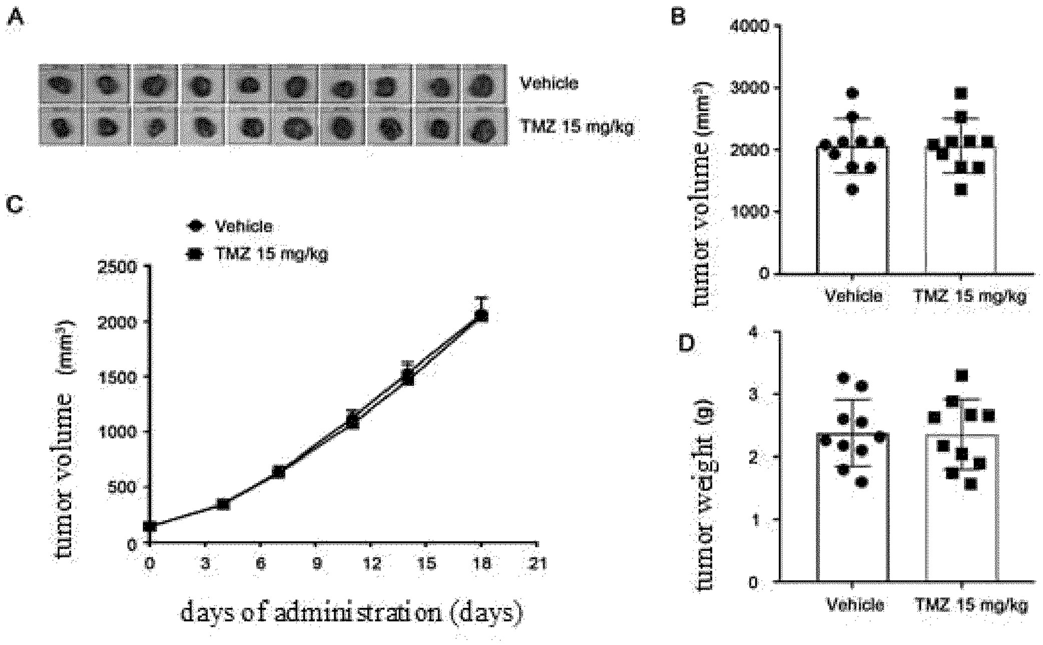
A
Vehicle
TMZ 15 mg/kg
B
tumor volume (mm³)
Vehicle
TMZ 15 mg/kg
C
Vehicle
TMZ 15 mg/kg
tumor volume (mm³)
days of administration (days)
D
tumor weight (g)
Vehicle
TMZ 15 mg/kg

FIG.12

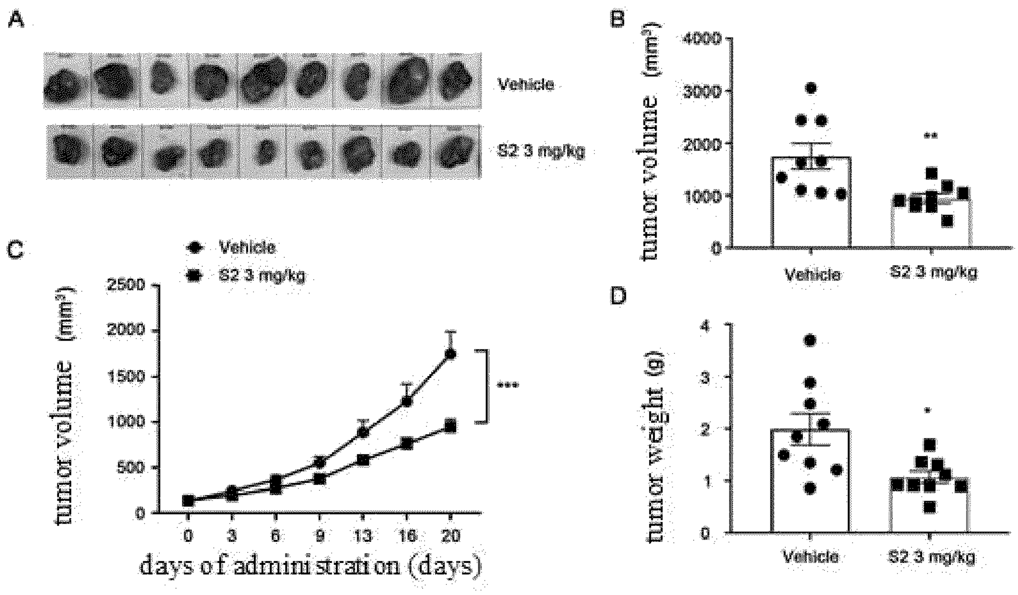
A
Vehicle
S2 3 mg/kg
B
tumor volume (mm³)
Vehicle
S2 3 mg/kg
C
Vehicle
S2 3 mg/kg
tumor volume (mm³)
days of administration (days)
D
tumor weight (g)
Vehicle
S2 3 mg/kg

FIG.13

**INTERNATIONAL SEARCH REPORT**

International application No.
**PCT/CN2022/133165**

**A. CLASSIFICATION OF SUBJECT MATTER**

A61K 31/4375(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

CNTXT; ENTXTC; VCN; CJFD; DWPI; CNKI; WFNPL; NPTXT; NPABS; Web of Science; STNext: 南京宁丹新药技术有限公司, 张正平, 二氢-萘啶, structural formula search, 肿瘤, 癌, 胶质瘤, Dihydro-naphthyridine, tumor, carcinoma, cancer, glioma

**C. DOCUMENTS CONSIDERED TO BE RELEVANT**

| Category* | Citation of document, with indication, where appropriate, of the relevant passages | Relevant to claim No. |
|---|---|---|
| A | CN 106632317 A (JIANGSU SIMOVAY PHARMACEUTICAL CO., LTD.) 10 May 2017 (2017-05-10)<br>description, abstract, and claim 3 | 1-10 |
| A | CN 103145704 A (JIANGSU SIMCERE PHARMACEUTICAL CO., LTD.) 12 June 2013 (2013-06-12)<br>entire document | 1-10 |
| A | 冯连顺等 (FENG, Lianshun et al.). "喹啉和萘啶衍生物抗肿瘤活性及其构-效关系研究进展 (Non-official translation: Research Progress of Anti-Tumor Activity and Configuration-Effect Relationship of Quinoline and Naphthyridine Derivatives)"<br>*国外医药抗生素分册 (World Notes on Antibiotics),*<br>Vol. 29, No. 6, 31 December 2008 (2008-12-31),<br>ISSN: ,<br>pages 268-274 and 282 | 1-10 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 January 2023** | **17 January 2023** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2022/133165**

| Patent document cited in search report | | | Publication date (day/month/year) | Patent family member(s) | | | Publication date (day/month/year) |
|---|---|---|---|---|---|---|---|
| CN | 106632317 | A | 10 May 2017 | WO | 2013083070 | A1 | 13 June 2013 |
| | | | | CN | 103145704 | A | 12 June 2013 |
| | | | | US | 2014364443 | A1 | 11 December 2014 |
| | | | | JP | 2015500250 | A | 05 January 2015 |
| | | | | CN | 104203945 | A | 10 December 2014 |
| | | | | AU | 2012349055 | A1 | 19 June 2014 |
| | | | | EP | 2789613 | A1 | 15 October 2014 |
| CN | 103145704 | A | 12 June 2013 | WO | 2013083070 | A1 | 13 June 2013 |
| | | | | US | 2014364443 | A1 | 11 December 2014 |
| | | | | CN | 106632317 | A | 10 May 2017 |
| | | | | JP | 2015500250 | A | 05 January 2015 |
| | | | | CN | 104203945 | A | 10 December 2014 |
| | | | | AU | 2012349055 | A1 | 19 June 2014 |
| | | | | EP | 2789613 | A1 | 15 October 2014 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202111599362
- CN 104203945 B
- CN 106632317 A

### Non-patent literature cited in the description


- *CA: a cancer journal for clinicians,* 2020, vol. 70 (4), 299-312
- *Oncogene,* 1999, vol. 18 (13), 2281-90
- *Journal of medicinal chemistry,* 2012, vol. 55 (5), 1940-56
- *The American journal of pathology,* 2014, vol. 184 (6), 1630-42
- *Molecular cancer research: MCR,* 2010, vol. 8 (10), 1358-74
- *Oncotarget,* 2016, vol. 7, 55863-55889
- *BMC cancer,* 2019, vol. 19, 77
- *BBA-Molecular Cell Research,* 2020, 118807
- *Journal of Medicinal Chemistry,* 2018, vol. 61 (12), 5424-5434